# EUROPEAN PATENT APPLICATION

(11) **EP 4 316 526 A1**
(43) Date of publication of application: **07.02.2024**
(21) Application number: 22779062.3
(22) Date of filing: 31.03.2022
(51) Int. Cl.: A61K 47/68, A61K 47/62, A61K 45/06, A61K 38/07, A61K 31/537, A61P 35/00, C40B 40/06

(54) **METHOD FOR CONSTRUCTION OF NUCLEIC ACID SELF-ASSEMBLY-MEDIATED ADC DRUG AND USE THEREOF**

(30) Priority: 01.04.2021 CN 202110354236
(71) Applicant: Assembly Medicine, LLC., Shanghai, 201203 (CN)
(72) Inventor: ZHOU, Liujuan, Shanghai 201203 (CN); CAO, Chan, Shanghai 201203 (CN); WANG, Nan, Shanghai 201203 (CN); PAN, Liqiang, Shanghai 201203 (CN); CHOU, James Jeiwen, Shanghai 201203 (CN)
(74) Representative: Lavoix
(86) International application number: PCT/CN2022/084311
(87) International publication number: WO 2022/206881

(57) **Abstract**

The present invention relates to the field of biotechnology drugs. In particular, provided in the present invention is an antibody-drug conjugate (ADC) complex based on a complementary and paired nucleic acid skeleton. The ADC complex is a polymer formed by means of compounding n monomers having complementary and paired nucleic acid skeletons, wherein the polymer contains: m "targeting monomers", which are cell-surface-targeting antibodies or proteins linked to nucleic acid single strands, and k "drug monomers", which are drugs (toxin payload) linked to nucleic acid single strands, n is a positive integer of 2-8, m is a positive integer of 1-3 and m < n, and k is a positive integer of 1-(n-m). In the polymer, each nucleic acid single strand of the monomer is complemented with a nucleic acid single strand of the other 1-3 monomers to form a complementary and paired double strand by means of base complementation, so that a complementary and paired nucleic acid skeleton structure is formed.

## Description

### TECHNICAL FIELD

The present invention relates to the field of biotechnology medicine, in particular to the method for construction of antibody-drug conjugate (ADC) mediated by nucleic acid polymerization and use thereof.

### BACKGROUND

ADC is a targeted drug developed for cancer treatment, which can specifically deliver chemotherapy drugs with strong cell cytotoxicity to cancer cells, reducing non-specific killing of cells in the human body. The mechanism of ADC is that it is endocytosed by the cell through the binding of antibodies to receptors on the surface of the cancer cell, then released in lysosomes, escaped from the lysosomes and entered the cytoplasm, leading to cell death. The concept of ADC has been proposed for a long time, but due to limitations in various knowledge and technical conditions, the development of related drugs was once slow and quite complicated. However, with the advancement of technology and the accumulation of clinical research experience, ADC drugs have gradually bottomed out in recent years and become one of the mainstream in the field of biopharmaceutical development.

At present, the preparation of ADC molecules in the field focuses on the coupling between the compounds (linker-payloads) formed by chemical linkers and small molecules and monoclonal antibody molecules. The classic method is to site-selectively couple the linker to lysine or cysteine site on the surface of the antibody molecule through specific chemical reactions. However, due to the fact that there are usually multiple lysine or cysteine sites on the antibody molecule and the steric hindrance effect near each amino acid is not entirely the same, the number of linker-payloads actually coupled to the antibody molecule, known as Drug-Antibody Ratio (DAR), is uneven, which brings great uncertainty to the analysis of the efficacy, pharmacokinetics, biological distribution, toxicology, and other aspects of ADC, and also leads to high production costs of ADC. However, because the uneven condition of DAR is originated from the inherent shortcomings of traditional coupling methods, even ADC molecules that have been approved by the FDA have highly heterogeneous DARs.

Therefore, there is an urgent need to develop a simple, flexible, efficient, and modular method for preparing ADC drugs in this field, which can provide ADC with a completely uniform DAR.

### SUMMARY OF THE INVENTION

The main purpose of the present invention is to provide a simple, flexible, efficient, and modular method for preparing ADC drugs, which can provide ADC with a completely uniform DAR.

In the first aspect of the present invention, it provides an antibody-drug conjugate (ADC) based on a complementary and paired nucleic acid skeleton, which is a polymer formed by the combination of n monomers with the complementary and paired nucleic acid skeleton, wherein the polymer comprises: m targeted monomers, wherein the "targeted monomer" is an antibody or protein that targets the cell surface and is connected to a nucleic acid single strand; and k drug monomers, wherein the "drug monomer" is a drug (toxin payload) connected to a nucleic acid single strand; wherein n is a positive integer of 2-8, m is a positive integer of 1-3 and m<n, and k is a positive integer of 1-(n-m); in the polymer, the nucleic acid single strand of each monomer forms a complementary double strand with the nucleic acid single strand of the other 1-3 monomers through complementary base pairing, thereby forming a complementary and paired nucleic acid skeleton structure.

In another preferred embodiment, n=3-8.

In another preferred embodiment, the targeted monomer has a structure as shown in Formula I:

A-W-D0 (I)

the drug monomer has a structure as shown in Formula II:

D1-W-D2 (II)

wherein,
A is an antibody or protein;
W is a single stranded nucleic acid sequence;
D0 is none or a drug;
D1 is none or a drug
D2 is none or a drug
"-" is a linker or bond,
at least one of D0, D1, and D2 is a drug.

In another preferred embodiment, the "-" is a covalent bond, a linker, or a combination thereof.

In another preferred embodiment, the A is an antibody or protein that specifically binds to cell surface receptors to cause endocytosis.

In another preferred embodiment, the antibody or protein is selected from the group consisting of: anti-HER2 antibody, anti-HSA antibody, anti-PD-L1 antibody, and combinations thereof.

In another preferred embodiment, the antibody or protein is an anti-HER2 nanobody, and the amino acid sequence thereof is as shown in SEQ ID NO: 1.

In another preferred embodiment, the D1/D2 is a small molecule or polypeptide toxin for killing cells.

In another preferred embodiment, the nucleic acid strand is resistant to degradation and selected from the group consisting of: L-nucleic acid, peptide nucleic acid, locked nucleic acid, phosphoromorpholidate nucleic acid, phosphorothioate modified nucleic acid, 2'- fluoro modified nucleic acid, 5-hydroxymethylcytosine nucleic acid, and combinations thereof.

In another preferred embodiment, in the polymer, the component A of each targeted monomer is the same or different.

In another preferred embodiment, in the polymer, the component D1 of each drug monomer is the same or different, and the component D2 of each drug monomer is the same or different.

In another preferred embodiment, in the polymer, the W of each monomer is different.

In another preferred embodiment, the nucleotides at both ends and/or in the middle of W may be chemically modified to link with the drug D1 or D2.

In another preferred embodiment, the single stranded nucleic acid sequence W in the targeted monomer (Formula I) and drug monomer (Formula II) has a structure as shown in Formula III:

X1-R1-X2-R2-X3 (III)

wherein,
R1 is a complementary base pairing region 1;
R2 is a complementary base pairing region 2;
X1, X2 and X3 are each independently none or redundant nucleic acid;
"-" is a bond.

In another preferred embodiment, the lengths of R1 and R2 are each independently 10-20 bases, preferably 14-16 bases.

In another preferred embodiment, the length of X1 is 0-5 bases.

In another preferred embodiment, the length of X3 is 0-5 bases.

In another preferred embodiment, the length of X2 is 0-3 bases.

In another preferred embodiment, the sequence of X2 is selected from the group consisting of: A, AA, AGA or AAA.

In another preferred embodiment, the R1 of each monomer forms a complementary base pairing structure with the R2 of the left neighboring (or left side) monomer; and the R2 forms a complementary base pairing structure with the R1 of the right neighboring (or right side) monomer.

In the second aspect of the present invention, it provides a pharmaceutical composition comprising:
(a) the antibody-drug conjugate based on a complementary and paired nucleic acid skeleton of the first aspect,
   wherein the targeted monomer is selected from a targeted monomer library; the drug monomer is selected from a drug monomer library; and
(b) a pharmaceutically acceptable carrier.

In another preferred embodiment, the pharmaceutical composition comprises a 2-8 polymer complex.

In another preferred embodiment, an assembly unit in the targeted monomer library has an antibody or protein that can specifically bind to cell surface receptors to cause endocytosis.

In another preferred embodiment, an assembly unit in the drug monomer library has a small molecule or polypeptide drug moiety that kills cells.

In another preferred embodiment, the targeted monomer and drug monomer in the pharmaceutical composition may be selected according to personalized treatment needs, and prepared into polymeric ADC drugs through real-time self-assembly.

In another preferred embodiment, the ratios of A/(D1+D2) and D1/D2 in the pharmaceutical composition may be selected according to personalized treatment needs.

In the third aspect of the present invention, it provides a nucleic acid sequence library, which comprises a nucleic acid sequence for forming the antibody-drug conjugate based on a complementary and paired nucleic acid skeleton of the first aspect.

In another preferred embodiment, the nucleic acid sequence W has a structure as shown in Formula III:

X1-R1-X2-R2-X3 (III)

wherein,
R1 is a complementary base pairing region 1;
R2 is a complementary base pairing region 2;
X1, X2 and X3 are each independently none or redundant nucleic acid;
"-" is a bond.

In the fourth aspect of the present invention, it provides use of the nucleic acid sequence library of the third aspect, for preparing the antibody-drug conjugate based on a complementary and paired nucleic acid skeleton of the first aspect.

In the fifth aspect of the present invention, it provides a method for preparing the antibody-drug conjugate of the first aspect, which comprises the steps of:
(1) forming a nucleic acid-drug assembly unit through chemical site-specific coupling of a nucleic acid single strand with a cytotoxic drug;
(2) forming an antibody-nucleic acid assembly unit through site-specific coupling of an antibody with a complementary nucleic acid single strand;
(3) forming the antibody-drug conjugate through rapid self-assembly of the antibody-nucleic acid assembly unit with multiple complementary nucleic acid-drug assembly units via complementary nucleic acid sequences.

It should be understood that within the scope of the present invention, each technical feature of the present invention described above and in the following (such as examples) can be combined with each other to form a new or preferred technical solution, which is not redundantly repeated one by one due to space limitations.

### DESCRIPTION OF THE DRAWINGS

Figure 1 shows the schematic diagram of ADC drug self-assembly mediated by complementary pairing of nucleic acid strands.
Figure 2 shows the gel electropherogram of anti-HER2 nanobodies purified by nickel column affinity chromatography.
Figure 3 shows the gel electropherogram of the coupling efficiency between anti-HER2 nanobodies and L-DNA.
Figure 4 shows the ion exchange purification of anti-HER2 nanobody-L-DNA conjugates.
Figure 5 shows the identification of DM4-L-DNA1 coupling efficiency by negative ion mode of ESI ion trap liquid chromatography-mass spectrometry.
Figure 6 shows the separation results of DM4-L-DNA1 by phenyl-agarose gel hydrophobic purification column.
Figure 7 shows the identification of MMAE-L-DNA1 coupling efficiency by negative ion mode of ESI ion trap liquid chromatography-mass spectrometry.
Figure 8 shows the gel electropherogram of self-assembly nanobody-drug conjugates, wherein a. SDS-PAGE; b. 2% agarose DNA gel.
Figure 9 shows the *in vitro* cytotoxicity evaluation of nanobody-drug conjugates.
Figure 10 shows the *in vitro* cytotoxicity evaluation of nanobody-drug conjugates in different tumor cell lines.
Figure 11 shows the changes in tumor volume of BALB/c-nu mice models inoculated subcutaneously with BT474 human breast cancer cells in different administration groups. The data are represented by Mean ± SEM. N=3-6 per group.
Figure 12 shows the terminal tumor weight of BALB/c-nu mice models inoculated subcutaneously with BT474 human breast cancer cells in different administration groups. The data are represented by mean ± SEM. Compared with the PBS group, independent sample T-test was used. N=3-6 per group.
Figure 13 shows the changes in body weight of BALB/c-nu mice models inoculated subcutaneously with BT474 human breast cancer cells in different administration groups. The average body weight is represented by mean ± SEM. N=3-6 per group.

### DETAILED DESCRIPTION

After extensive and in-depth research, the present inventors unexpectedly developed an ADC complex formed by self-assembly based on complementary pairing of nucleic acid strands, as well as its preparation method and use thereof for the first time. Based on the characteristics of self-assembly mentioned above, the inventors have developed for the first time an ADC drug assembly unit library that can be used for instant preparation of ADC complexes. By using this preparation method, the assembly unit library can be used to quickly and efficiently prepare ADC drugs with low-cost and high-yield according to personalized treatment needs, which exhibit high-specific targeting and have highly uniform DARs. On this basis, the present invention has been completed.

Specifically, the present invention provides a multivalent protein drug, comprising n protein drug units, wherein each drug unit comprises a drug element moiety of the same kind and different nucleic acid element moieties connected to the drug element moiety; n is a positive integer ≥ 2; n different nucleic acid element moieties form a n-polymer through complementary nucleic acid bases, thereby forming the multivalent protein drug. The multivalent protein drug of the present invention can form a stable pairing structure simply through rapid assembly of complementary nucleic acid bases (such as within 1 minute), rather than complex peptide bonds or other chemical modifications, etc. Experiments have shown that the drug of the present invention can increase its molecular weight through multimerization, thereby extending its half-life in animals.

Specifically, the present invention provides an ADC composite drug based on a complementary and paired nucleic acid skeleton, which comprises n units that can accurately complete self-assembly to form an n-polymer complex; wherein n is a positive integer of 2; wherein the m targeted monomers are antibodies or proteins that target the cells and are connected to nucleic acid single strands, and k drug monomers are drugs (toxin payloads) connected to nucleic acid single strands, while n is a positive integer of 3-8, m (<n) is a positive integer of 1-4, and k is a positive integer of 1-(n-m); in the polymer, the nucleic acid single strand of each monomer forms a complementary double strand with the nucleic acid single strand of the other 1-3 monomers through complementary base pairing, thereby forming the n-polymer ADC complex. The ADC composite drug of the present invention can form a stable pairing structure simply through rapid assembly of complementary nucleic acid bases (such as within 1 minute), while having a uniform DAR (whereas the ADC drug obtained through random chemical modification does not). Experiments have shown that the ADC composite drug of the present invention can specifically target cells to cause endocytosis, thereby killing the cells.

### Term

As used herein, the terms "the conjugate of antibody and drug of the present invention", "the antibody-drug conjugate of the present invention", "the conjugate of the present invention", "the antibody-conjugated drug of the present invention", "polymeric ADC drug", "the ADC complex based on a complementary and paired nucleic acid skeleton of the present invention", "ADC of the present invention", or "ADC drug of the present invention" can be interchangeably used and refer to ADC complexes with the structure shown in Formula I.

Unless otherwise defined, all technical and scientific terms used herein have the same meanings as those commonly understood by those skilled in the art to which the present invention belongs.As used herein, when used in reference to specific enumerated values, the term "about" means that the value may vary by no more than 1% from the enumerated value. For example, as used herein, "about 100" includes all values between 99 and 101 (e.g., 99.1, 99.2, 99.3, 99.4, etc.).

### ADC

In the present invention, the ADC refers to Antibody-drug conjugates, comprising an antibody that can specifically target cell surface molecules, a drug with cytotoxicity, and a linker bridging the antibody and drug.

Typically, the antibody in the ADC includes, but is not limited to, a nanobody, a single chain antibody, an Fab, a monoclonal antibody, etc..

Typically, the drug in the ADC includes, but is not limited to, MMAE/MMAF, DM1/DM4, Caliheamicin, Duocarimycin, PBD, amanitin, SN38, DXd, PNU-159682, etc..

In a preferred embodiment of the present invention, the antibody is an anti-HER2 single domain antibody, the drug is DM4 or MMAE, and the linker is complementary nucleic acid single strands.

### Targeted antibody/protein A

In the present invention, the antibody/protein element moiety can specifically target cell surface molecules and cause endocytosis.

Typically, the antibody/protein includes but is not limited to a nanobody, a single chain antibody, an Fab, a monoclonal antibody, a cytokine, a hormone (such as insulin, growth hormone, etc.), a peptide.

In a preferred embodiment of the present invention, the antibody element moiety is an anti-HER2 single domain antibody, which is used to target HER2 receptors on BT474 cells. The amino acid sequence of the anti-HER2 single domain antibody is shown in SEQ ID NO: 1.

In another preferred embodiment of the present invention, the antibody element moiety further comprises an anti-HSA antibody. The amino acid sequence of the anti-HSA antibody is as shown in SEQ ID NO: 2.

SEQ ID NO: 2, the amino acid sequence of anti-HSA nanobody mutant:

MGSAHHHHHHWSHPQFEKGGGSGGGSGGSAWSHPQFEKENLYFQSAVQLVES
GGGLVQPGNSLRLSCAASGFTFRSFGMSWVRQAPGKEPEWVSSISGSGSDTLYA
DSVKGRFTISRDNAKTTLYLQMNSLKPEDTAVYYCTIGGSLSRSSQGTQVTVSS
GSC

### Drug D

In the present invention, the drug element moiety is a small molecule or polypeptide drug payload commonly used in ADC drugs, which can enter the lysosome and then be released into the cytoplasm after the targeted monomer-drug monomer complex is endocytosed by the cell.

Typically, the drug element moiety includes, but is not limited to, microtubule toxins such as auristatins, maytaninoids, epothilone, toxoids, tubulysins and vinorelbine; DNA toxins such as calicheamicins, duoarmycins and analogs, PBD-dimers, doxorubicin, topotecan, blemycin A2, dactinomycin and mitomycin C; transcriptional toxins such as amatoxins and thalanstatin A; inhibitors such as oligomycin and ipatasertib.

In a preferred embodiment of the present invention, the drug element moiety is MMAE, which acts on microtubule proteins and disrupts the microtubule network of the cell, leading to cell cycle arrest and apoptosis.

### Nucleic acid chain W

As used herein, the terms "nucleic acid strand", "nucleic acid single strand", "single stranded nucleic acid", and "nucleic acid element moiety" can be interchangeably used, all referring to the complementary and paired nucleic acid skeleton structure forming the antibody-drug conjugate of the present invention, which is a segment of nucleic acid sequence connected with the antibody, protein, or drug (toxin payload).

In the present invention, the nucleic acid chain is resistant to biodegradation in the body and does not cause a strong natural immune response.

Typically, the nucleic acid element moiety includes, but is not limited to, L-nucleic acid, peptide nucleic acid, locked nucleic acid, phosphoromorpholidate nucleic acid, phosphorothioate modified nucleic acid, 2'-fluoro modified nucleic acid, 5-hydroxymethylcytosine nucleic acid.

In a preferred embodiment of the present invention, the nucleic acid element moiety is four L-DNA with different sequences that can accurately self-assemble into a stable tetramer nucleic acid skeleton. Left-handed nucleic acid refers to a mirror image of the natural right-handed nucleic acid (D-nucleic acid), which can be divided into left-handed DNA (L-DNA) and left-handed RNA (L-RNA). Left-handed (chiral center) mainly exists in the deoxyribose or ribose portion of nucleic acids, exhibiting mirror flipping. Therefore, left-handed nucleic acids cannot be degraded by ubiquitous nucleases in the plasma, such as exonucleases and endonucleases.

### Preparation method

The present invention also provides a method for preparing an ADC (antibody-drug conjugate) complex based on a complementary nucleic acid skeleton, comprising the steps of:

### 1. Design and Preparation of L-Nucleic Acid Chain Framework

According to the present invention, the L-nucleic acid chain framework is formed by base pairing of two or more L-nucleic acid single strands. The 5' or 3' end of each L-nucleic acid single strand is activated into functional groups that can be subsequently modified (such as NH2), and then one end of the linker (such as SMCC, SBAP) is coupled with the activated functional group on the L-nucleic acid single strand. L-nucleic acids with linkers can be assembled into the desired L-nucleic acid chain framework. In another preferred embodiment, the activated functional groups (such as aldehide, maleimide) at the 5' or 3' end of the L-nucleic acid single strand are already included during nucleic acid synthesis. After confirming that the L-nucleic acids with linkers can successfully self-assemble a framework, the L-nucleic acid single strands with linkers can be coupled with antibodies respectively for subsequent assembly. Basically, the L-nucleic acid framework of the present invention can be prepared by the following steps.

### 1.1 Design of L-nucleic acid single strand for rapid self-assembly

The required number of multivalent (n) (such as trimers, tetramers) is determined. The number of required L-nucleic acid single strands (n) is determined based on the multivalent number n. Corresponding number of L-nucleic acid single strand sequences are designed, and the stability of the target nucleic acid framework is regulated by optimizing base pairing to reduce the possibility of non-specific pairing between nucleic acid chains. The details of nucleic acid sequence design are specifically described in the summary of the invention and examples.

In a preferred embodiment of the present invention, the following L-DNAs are prepared:
L-DNA1: 5' AGGCGATCACAATCCAAATGAGCGTGTTACGG 3' (SEQ ID NO: 4)
L-DNA2: 5' ACCGTAACACGCTCAAAACCGAAGTGCCAATT 3' (SEQ ID NO: 5)
L-DNA3: 5' AAATTGGCACTTCGGAAAACTATGCGGCTGCT 3' (SEQ ID NO: 6)
L-DNA4: 5' AAGCAGCCGCATAGTAAAGGATTGTGATCGCC 3' (SEQ ID NO: 7)

### 1.2 Activation of L-DNA or L-RNA

The activation of L-nucleic acid involves modification of active groups at the 5' (X1) or 3' (X3) end thereof and subsequent conjugation with linkers. The modification of active groups can be customized in nucleic acid synthesis companies. The linker generally has bifunctional groups, that is, one end can be coupled with the active group of nucleic acid, and the other end can be connected to a specific site on the protein (such as NH₃, SH).

According to a preferred embodiment of the present invention, all L-nucleic acids forming the framework are modified by adding NH₂ at the 5' end, thereby completing the activation of L-nucleic acids and allowing it to be subsequently coupled to the thiol groups on the cysteine of the protein.

### 2. Preparation method of protein-L-nucleic acid complex

Firstly, the 5' or 3' end of the L-nucleic acid is modified with an active group (such as NH₂), and then another active group is introduced at a specific site of the protein (such as introducing a cysteine mutation at the C-terminus of the protein). Then, a double linker molecule (such as SMCC) is used to bridge the L-nucleic acid and the protein, and the protein-L-nucleic acid complex is purified through affinity chromatography, ion exchange chromatography, and other methods.

### 3. Preparation method of drug-L-nucleic acid complex

The L-nucleic acid is modified by adding NH₂ to the 5' end thereof, for activation and subsequent connection of the L-nucleic acid. Drugs generally carry -SuO/-NHS reaction groups, which can be coupled with the active groups of nucleic acids.

### The main advantages of the present invention include:

(1) The present invention can flexibly and efficiently prepare polymer ADC complex drugs, and can prepare personalized ADC drugs in a short time according to the requirements of the target and the combination of drug payloads, for personalized treatment;
(2) the preparation of ADC drugs of the present invention can easily achieve highly uniform DAR;
(3) the present invention can easily achieve the preparation of dual- or even triple- specific ADC drugs, while ensuring highly uniform DAR, by utilizing the characteristics of modular and precise self-assembly;
(4) the present invention can easily achieve the preparation of ADC drugs with two different drug payloads, while ensuring highly uniform DAR, by utilizing the characteristics of modular and precise self-assembly, for solving the problem of ADC resistance.

The present invention is further explained below in conjunction with specific example. It should be understood that these examples are only for illustrating the present invention and not intend to limit the scope of the present invention. The conditions of the experimental methods not specifically indicated in the following examples are usually in accordance with conventional conditions, such as those described in Sambrook et al., Molecular Cloning: A Laboratory Manual (New York: Cold Spring Harbor Laboratory Press, 1989), or according to the conditions recommended by the manufacturer. Unless otherwise stated, percentages and parts are calculated by weight.

### Example 1: Preparation of Anti-HER2 nanobody

The His tag and double Strep tags were added to the amino end of the nanobody for protein purification, and cysteine mutations were introduced at the carboxyl end of the nanobody for nucleic acid site-specific coupling. The gene sequence of the anti-HER2 nanobody was optimized to the preferred codons of *Escherichia coli,* and then subcloned into the pET-28b (+) plasmid. The amino acid sequence of the anti-HER2 nanobody is SEQ ID NO: 1.

SEQ ID NO: 1, the amino acid sequence of anti-HER2 nanobody mutant:

MSAHHHHHHWSHPQFEKGGGSGGGSGGSAWSHPQFEKENLYFQSEVQLV
ESGGGLVQAGGSLRLSCAASGITFSINTMGWYRQAPGKQRELVALISSIGDTYY
ADSVKGRFTISRDNAKNTVYLQMNSLKPEDTAVYYCKRFRTAAQGTDYWGQG
TLVTVSSGSC

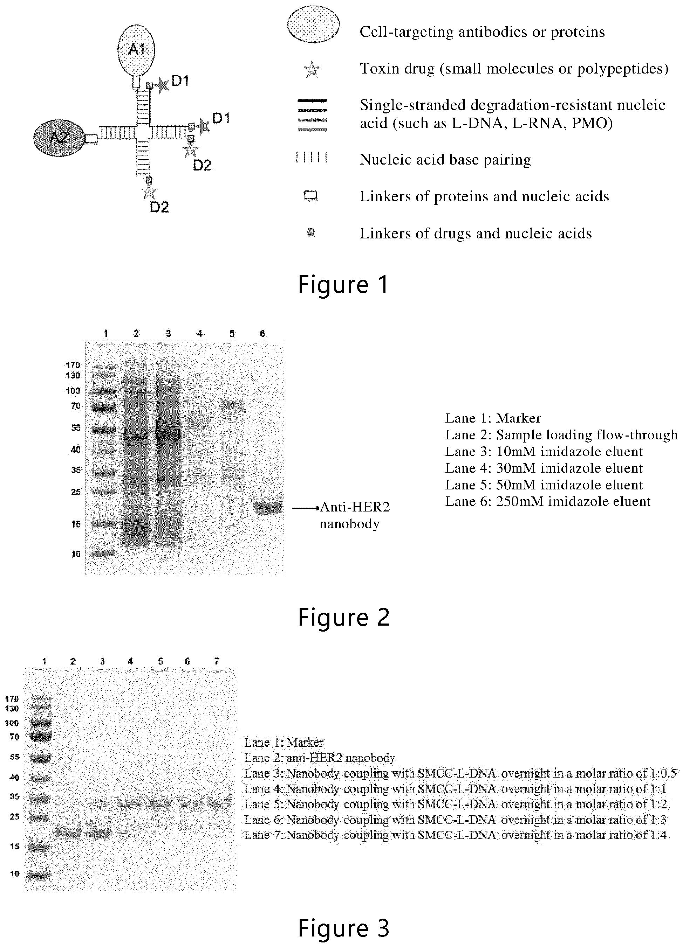

1 ul of the constructed expression vector was taken and transformed into *Escherichia coli* shuffle T7. The transformed single colony of shuffle T7 was selected and transfered to LB medium (containing 50 ug/mL kanamycin) and incubated at 37 °C until OD600 = 0.6-0.8. IPTG at a final concentration of 0.1 mM was added to induce expression. The incubation was continued at 16 °C for 18 to 20 hours. The bacteria cells after expression were collected by centrifugation, and resuspended in Tris buffer (20 mM Tris HCl, 200mM NaCl, pH 7.4), and added with a small amount of reducing agent (such as 10 µM TCEP) and protease inhibitor cocktail (Sigma). The bacteria were broken by ultrasonication, and the bacterial solution was centrifuged at 17,000 rpm for 30 minutes before collecting the supernatant. Nanobodies in the supernatant were purified using His tag affinity column. After the supernatant passed through the column, the column was washed with Tris buffer (50 mM Tris HCl, 200mM NaCl, 10 µM TCEP, pH 7.4) containing 10 mM, 30 mM, and 50 mM imidazole until the impurities no longer flow out (the filtrate was collected and the absorption of A280 of the filtrate was detected using UV to determine whether the impurities had been cleared). The nanobodies bound to the column were eluted with Tris buffer (50 mM Tris HCl, 200mM NaCl, 10 µ M TCEP, pH 7.4) containing 250 mM imidazole. High-purity nanobodies were obtained ultimately (Figure 2).

### Example 2: Coupling and purification of nanobody-nucleic acid conjugate

The anti-HER2 nanobodies purified in Example 1 were mixed with excess 1-2 times molar ratio of SMCC-L-DNA single strand (the specific molar ratio could be measured through pre-experiments) and placed at 4 °C for coupling reaction overnight. The coupling efficiency could reach over 90% (Figure 3).

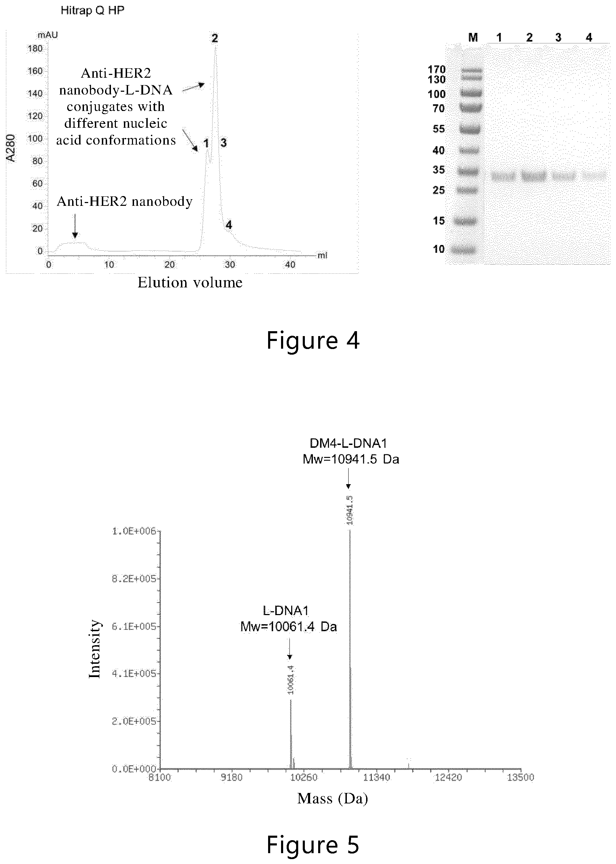

The unreacted SMCC-L-DNA single strands were removed by using Strep affinity column, and the mixture of nanobodies and nanobody-L-DNA was collected. The buffer of the mixture was replaced with the sample loading buffer of the anion exchange column. By taking advantage of the negatively charged nature of DNA, anion exchange column (HiTrap Q HP column) was used to further separate and purify nanobody-L-DNA and remove unreacted nanobodies. The separation process was achieved through gradient elution. The sample loading buffer is 20 mM Tris HCl, 15 mM NaCl, pH 8.5, and the elution buffer is 20 mM Tris HCl, 1 M NaCl, pH 8.5. The gradient elution was performed with 0-100% elution buffer, and the unreacted nanobodies and nanobody-L-DNA peaked successively. Due to the presence of multiple conformations of DNA in the elution buffer, three elution peaks of nanobody-L-DNA conjugates appeared during gradient elution (Figure 4). Nanobody-L-DNA was collected. After concentration, the buffer was replaced with 50 mM NaH₂PO₄, 150 mM NaCl, pH 7.4 using a PD-10 desalination column.

### Example 3: Coupling of toxin DM4-nucleic acid conjugate

The coupling of DM4-L-DNA1 is taken as an example. Firstly, SPDB-DM4 powder was dissolved in 100% dimethyl acetamide to obtain SPDB-DM4 solution at a final concentration of 10 mM. L-DNA1 was dissovled in 100% phosphate buffer to obtain L-DNA1 solution at a final concentration of 1 mM. Then 1 mM L-DNA1 solution, 10mM SPDB-DM4 solution, and phosphate reaction solution were mixed in a volume ratio of 2:5:3 for overnight reaction. The coupling efficiency could reach about 70%. The reaction products can be detected and analyzed by anion mode ESI ion trap liquid chromatography-mass spectrometry (Figure 5).

### Example 4: Purification of toxin DM4-nucleic acid conjugate

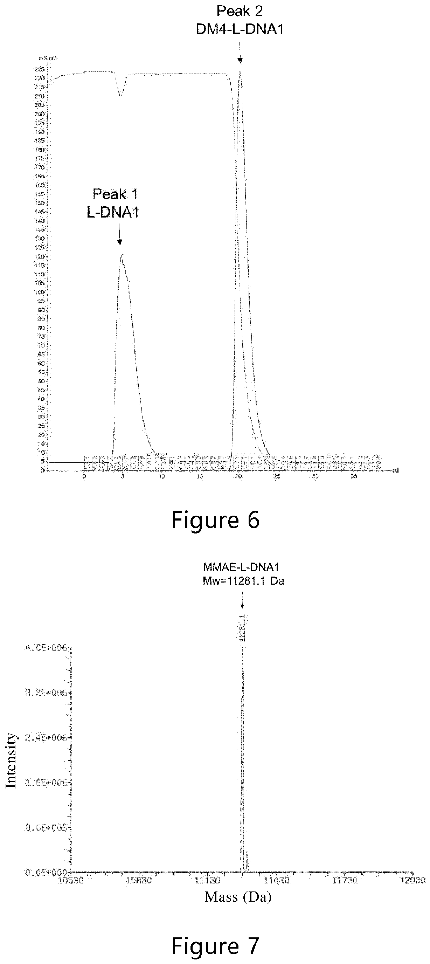

The purification of DM4-L-DNA1 is taken as an example. The reaction product of DM4-L-DNA1 was loaded on the phenyl agarose gel hydrophobic purification column. L-DNA1 and DM4-L-DNA1 could be completely separated by one-step elution with 20% ethanol (Figure 6). The separated product could be identified by anion mode ESI ion trap liquid chromatography-mass spectrometry.

### Example 5: Coupling of toxin MMAE-nucleic acid conjugate

The coupling of MMAE-L-DNA1 is taken as an example. Firstly, SuO-vc-PAB-MMAE powder was dissolved in 100% dimethyl acetamide to obtain SuO-vc-PAB-MMAE solution at a final concentration of 10mM. L-DNA1 was dissovled in 100% phosphate buffer to obtain L-DNA1 solution at a final concentration of 1mM. Then 1mM L-DNA1 solution, 10mM SuO-vc-PAB-MMAE solution, and phosphate reaction solution were mixed in a volume ratio of 2:5:3 for overnight reaction. The coupling efficiency was detected by anion mode ESI ion trap liquid chromatography-mass spectrometry, which could reach about 100% (Figure 7). The reaction product was precipitated with 100% ethanol and could be completely separated from the unreacted SuO-vc-PAB-MMAE. After being washed with 75% ethanol four times, the precipitate was dissolved in phosphate buffer.

### Example 6: Self-assembly of nanobody drug conjugate

The molecular structure of NAPPA₄-DM4_{(1,2)}-HER2_{(3,4)} of double antibodies and double toxins is taken as an examplebelow to introduce the self-assembly process of nanobody drug conjugate. DM4 is coupled to DNA 1 and 2, and anti-HER2 nanobodies (prepared in Example 1) are coupled to DNA 3 and 4.

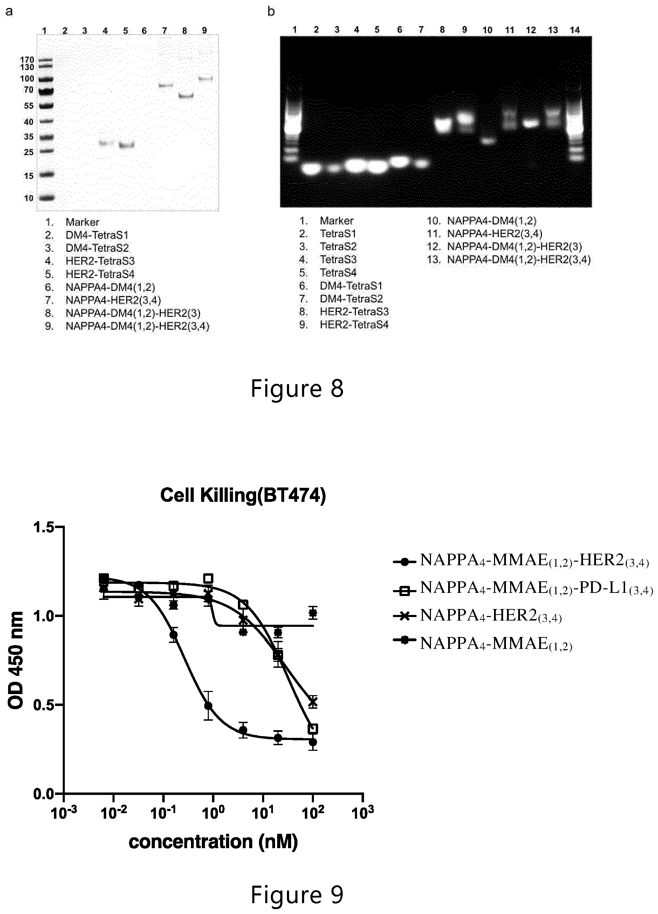

The concentrations of DM4-L-DNA1, DM4-L-DNA2, anti-HER2 Nb-L-DNA3 conjugate, and anti-HER2 Nb-L-DNA4 conjugate were measured, respectively. An appropriate amount of the above components were taken and preheated at 37 °C for 5 minutes, then mixed in a 1:1 molar ratio at 37 °C and incubated for 1 minute to complete the self-assembly of the nanobody drug conjugate with a molecular structure of NAPPA₄-DM4_{(1,2)}-HER2_{(3,4)} (Figure 8).

### Example 7: In vitro cell killing experiment of antibody drug conjugate based on L-nucleic acid framework

In order to further analyze the *in vitro* cytotoxicity of antibody-drug conjugate based on L-nucleic acid framework, four drug molecules, NAPPA₄-MMAE_{(1,2)}-HER2_{(3,4)}, NAPPA₄-MMAE_{(1,2)}-PD-L1_{(3,4)}, NAPPA₄-MMAE_{(1,2)}, and NAPPA₄-HER2_{(3,4)}, were assembled and evaluated for *in vitro* cytotoxicity using HER2 positive cell line BT474 as a cell model. Among them, the used anti-HER2 nanobody is the anti-HER2 nanobody prepared in the Example, and the amino acid sequence of the used anti-PD-L1 antibody is as shown in SEQ ID NO: 3.

SEQ ID NO: 3, the amino acid sequence of anti-PD-L1 nanobody mutant:

MGSAHHHHHHWSHPQFEKGGGSGGGSGGSSAWSHPQFEKENLYFQSEVQLLE
SGGGEVQPGGSLRLSCAASGGIFAIKPISWYRQAPGKQREWVSTTTSSGATNYA
ESVKGRFTISRDNAKNTLYLQMSSLRAEDTAVYYCNVFEYWGQGTLVTVKPGS
C

BT474 cells were inoculated onto a 96 well plate with a cell density of 20,000 cells per well. Triple wells were set and incubated in a 37 °C incubator. After 24 hours, culture medium was replaced with fresh one and drugs with specific concentration gradients were added, and continued to incubate in a 37 °C incubator. After 48 to 72 hours, culture medium was replaced with fresh one and added with 10% CCK8 solution, continued to incubate at 37°C in dark. After 2-4 hours, the absorbance of each sample at 450nm was measured using microplate reader, and the cytotoxicity of drugs to cells was calculated.

The experimental results show that compared to the control group NAPPA₄-MMAE_{(1,2)}-PD-L1_{(3,4)}, the target drug NAPPA₄-MMAE_{(1,2)}-HER2_{(3,4)} has a more significant killing effect on HER2 positive cell line BT474. However, NAPPA₄-MMAE_{(1,2)} and NAPPA₄-HER2_{(3,4)} have almost no killing effect (Figure 9).

In order to further validate the *in vitro* killing effect of antibody-drug conjugates based on L-nucleic acid framework on different tumor cell lines, two drug molecules, NAPPA₄-MMAE_{(1,2)}-HER2₍₃₎-HSA₍₄₎ and NAPPA₄-MMAE_{(1,2)}-HSA₍₄₎, were selected for *in vitro* cell killing of BT474, SK-BR-3, NCI-N87, HCC1954, SKOV-3, and Calu-3 tumor cell lines. The detection kit used was CellTier-Glo (Promega, G7572). The absorbance of each sample at 450nm was measured using microplate reader, and the cytotoxicity of drugs to cells was calculated.

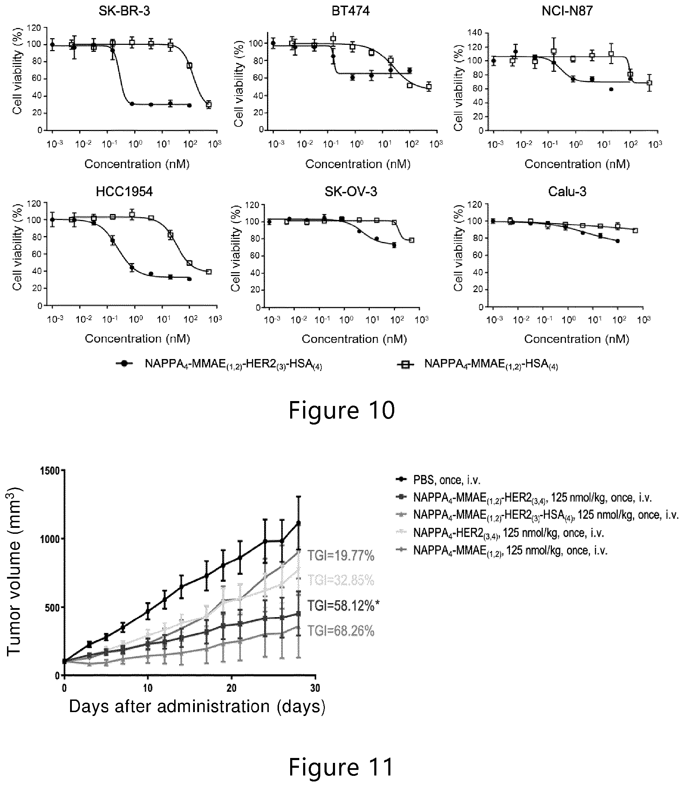

The experimental results show that compared to NAPPA₄-MMAE_{(1,2)}-HSA₍₄₎, NAPPA₄-MMAE_{(1,2)}-HER2₍₃₎-HSA₍₄₎ has a more significant killing effect on all six tumor cell lines, and the killing effect is positively correlated with the reported HER2 expression on the surface of tumor cells (Figure 10).

### Example 8: In vivo tumor inhibition experiment of antibody-drug conjugate based on L-nucleic acid framework

In order to test the *in vivo* tumor inhibitory activity of antibody-drug conjugate based on L-nucleic acid framework, a BT474 human breast cancer nude mouse model was constructed to evaluate the *in vivo* tumor inhibitory activity of drugs NAPPA₄-MMAE_{(1,2)}-HER2_{(3,4)} and NAPPA₄-MMAE_{(1,2)}-HER2₍₃₎-HSA₍₄₎, and NAPPA₄-MMAE_{(1,2)}, NAPPA₄-HER2_{(3,4)} and PBS groups were set as controls.

BT474 cells were cultured in DMEM medium containing 10% FBS and maintained in a saturated humidity incubator at 37 °C with 5% CO₂. BT474 cells in logarithmic growth phase were collected, resuspended in DMEM basal medium, and added with matrix gel at 1:1. Under sterile conditions, 0.2 mL of cell suspension was inoculated subcutaneously into the right back of nude mice at a concentration of 1 × 10⁷ cells/0.2 mL/mouse. The diameter of the xenograft tumor was measured with a vernier caliper, and the animals were randomly divided into groups of 6 in each group when the tumor grew to 100-300 mm³. The grouping day was defined as D0 day, and mice were administered with a single intravenous injection of the tested drugs NAPPA₄-MMAE_{(1,2)}-HER2_{(3,4)}, NAPPA₄-MMAE_{(1,2)}-HER2₍₃₎-HSA₍₄₎, NAPPA₄-MMAE_{(1,2)}, NAPPA₄-HER2_{(3,4)}, and PBS control at a dose of 125 nmol/kg. After starting the administration, the tumor size was measured with a vernier caliper and the weight of the mice was weighed on days D0, D3, D5, D7, D10, D12, D14, D17, D19, D21, D24, D26, and D28.

The calculation method for tumor volume is: tumor volume (mm³) = 0.5 × tumor long diameter × tumor short diameter².

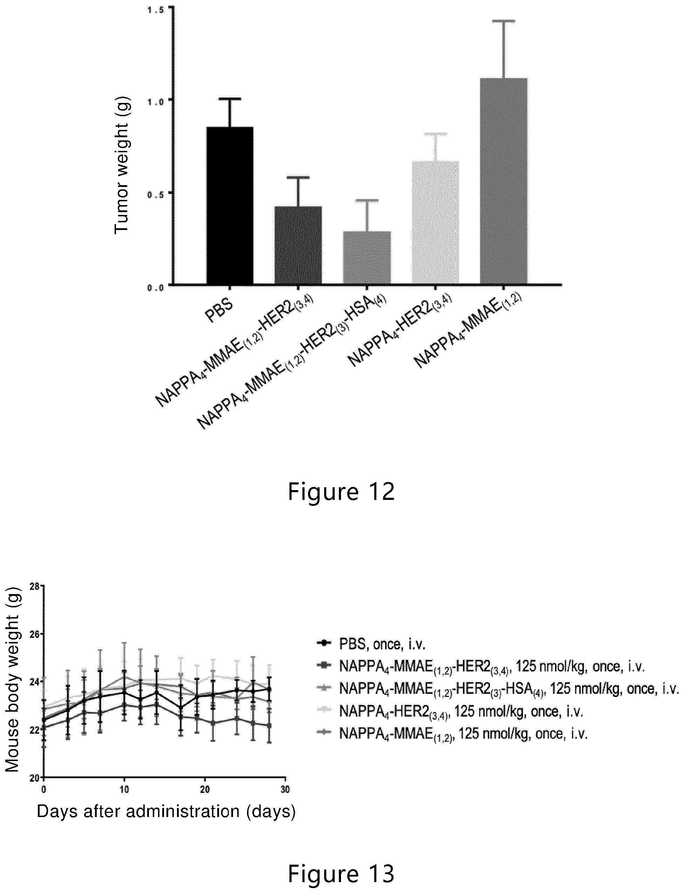

The average tumor volume changes of different groups of mice during the administration period (Figure 11) and the average tumor weight of different groups of mice at the end point of the experiment (Figure 12) show that compared to the PBS control group, NAPPA₄-MMAE_{(1,2)}-HER2_{(3,4)} and NAPPA₄-MMAE_{(1,2)}-HER2₍₃₎-HSA₍₄₎ have significant *in vivo* tumor inhibitory effects. And the drug containing anti-HSA antibody, NAPPA₄-MMAE_{(1,2)}-HER2₍₃₎-HSA₍₄₎, has a better tumor inhibitory effect *in vivo* than the drug NAPPA₄-MMAE_{(1,2)}-HER2_{(3,4)}, which does not contain anti-HSA antibody. The drugs NAPPA₄-MMAE_{(1,2)} and NAPPA₄-HER2_{(3,4)} have almost no killing effect. During the entire experimental observation period, the average body weight changes of different groups of mice remained within the normal fluctuation range (Figure 13), indicating that under a single administration dose of 125 nmol/kg, each test drug has no significant effect on the body weight of mice.

All references mentioned in the present application are incorporated by reference herein, as though individually incorporated by reference. In addition, it should be understood that after reading the above teaching content of the present invention, various changes or modifications may be made by those skilled in the art, and these equivalents also fall within the scope as defined by the appended claims of the present application.

## Claims

1. An antibody-drug conjugate (ADC) based on a complementary and paired nucleic acid skeleton, which is a polymer formed by the combination of n monomers with the complementary and paired nucleic acid skeleton, wherein the polymer comprises: m targeted monomers, wherein the "targeted monomer" is an antibody or protein that targets the cell surface and is connected to a nucleic acid single strand; and k drug monomers, wherein the "drug monomer" is a drug (toxin payload) connected to a nucleic acid single strand; wherein n is a positive integer of 2-8, m is a positive integer of 1-3 and m<n, and k is a positive integer of 1-(n-m); in the polymer, the nucleic acid single strand of each monomer forms a complementary double strand with the nucleic acid single strand of the other 1-3 monomers through complementary base pairing, thereby forming a complementary and paired nucleic acid skeleton structure.

2. The antibody-drug conjugate of claim 1, wherein the targeted monomer has a structure as shown in Formula I:
A-W-D0 (I)
the drug monomer has a structure as shown in Formula II:
D1-W-D2 (II)
wherein,
A is an antibody or protein;
W is a single stranded nucleic acid sequence;
D0 is none or a drug;
D1 is none or a drug
D2 is none or a drug
"-" is a linker or bond,
at least one of D0, D1, and D2 is a drug.

3. The antibody-drug conjugate of claim 2, wherein the A is an antibody or protein that specifically binds to cell surface receptors to cause endocytosis.

4. The antibody-drug conjugate of claim 2, wherein the D1/D2 is a small molecule or polypeptide toxin for killing cells.

5. The antibody-drug conjugate of claim 2, wherein the nucleic acid single strand is resistant to degradation and selected from the group consisting of: L-nucleic acid, peptide nucleic acid, locked nucleic acid, phosphoromorpholidate nucleic acid, phosphorothioate modified nucleic acid, 2'- fluoro modified nucleic acid, 5-hydroxymethylcytosine nucleic acid, and combinations thereof.

6. The antibody-drug conjugate of claim 2, wherein the single stranded nucleic acid sequence W in the targeted monomer (Formula I) and drug monomer (Formula II) has a structure as shown in Formula III:
X1-R1-X2-R2-X3 (III)
wherein,
R1 is a complementary base pairing region 1;
R2 is a complementary base pairing region 2;
X1, X2 and X3 are each independently none or redundant nucleic acid;
"-" is a bond.

7. The antibody-drug conjugate of claim 6, wherein the lengths of R1 and R2 are each independently 10-20 bases, preferably 14-16 bases.

8. The antibody-drug conjugate of claim 6, wherein the R1 of each monomer forms a complementary base pairing structure with the R2 of the left neighboring (or left side) monomer; and the R2 forms a complementary base pairing structure with the R1 of the right neighboring (or right side) monomer.

9. A pharmaceutical composition comprising:
(a) the antibody-drug conjugate based on a complementary and paired nucleic acid skeleton of claim 1,
wherein the targeted monomer is selected from a targeted monomer library; the drug monomer is selected from a drug monomer library; and
(b) a pharmaceutically acceptable carrier;
wherein, an assembly unit in the targeted monomer library has an antibody or protein that can specifically bind to cell surface receptors to cause endocytosis; and an assembly unit in the drug monomer library has a small molecule or polypeptide drug moiety that kills cells.

10. A nucleic acid sequence library, which comprises a nucleic acid sequence for forming the antibody-drug conjugate based on a complementary and paired nucleic acid skeleton of claim 1.

11. The nucleic acid sequence library of claim 10, wherein the nucleic acid sequence has a structure as shown in Formula III:
X1-R1-X2-R2-X3 (III)
wherein,
R1 is a complementary base pairing region 1;
R2 is a complementary base pairing region 2;
X1, X2 and X3 are each independently none or redundant nucleic acid;
"-" is a bond.

12. Use of the nucleic acid sequence library of claim 10 for preparing the antibody-drug conjugate based on a complementary and paired nucleic acid skeleton of claim 1.

13. A method for preparing the antibody-drug conjugate of claim 1, which comprises the steps of:
(1) forming a nucleic acid-drug assembly unit through chemical site-specific coupling of a nucleic acid single strand with a cytotoxic drug;
(2) forming an antibody-nucleic acid assembly unit through site-specific coupling of an antibody with a complementary nucleic acid single strand;
(3) forming the antibody-drug conjugate through rapid self-assembly of the antibody-nucleic acid assembly unit with multiple complementary nucleic acid-drug assembly units via complementary nucleic acid sequences.
